# EUROPEAN PATENT APPLICATION

(11) **EP 2 193 822 A1**
(43) Date of publication of application: **09.06.2010**
(21) Application number: 08021272.3
(22) Date of filing: 08.12.2008
(51) Int. Cl.: A61N 1/36

(54) **Bladder sphincter pacemaker**

(71) Applicant: Nduka, Harry, Barking London IG11 0PY (GB)
(72) Inventor: Nduka, Harry, Barking London IG11 0PY (GB)

(57) **Abstract**

This device will be able to ascertain that the intra vesical pressure (ie intrabladder) is risen (similar to pressure sensors used in urodynamic studies) and then stimulate the fast twitch muscle part of the sphincter complex to contract and stop incontinence. The slow acting part of the sphincter complex is usually working well stopping continous leakage so does not need to be stimulated this is the main second difference with the ideas proposed by others.

## Description

The electrode has a guide wire which is directly connected to the fast acting part of the sphincter. This is easy to localise using magnetic resonance imaging.

The pressure guage sensors are to sense the intra bladder pressure and once a pre set level is reached contraction is stimulated to stop urine leakage.

To pass urine normally the system is de activated temporarily using the radio frequency remote.

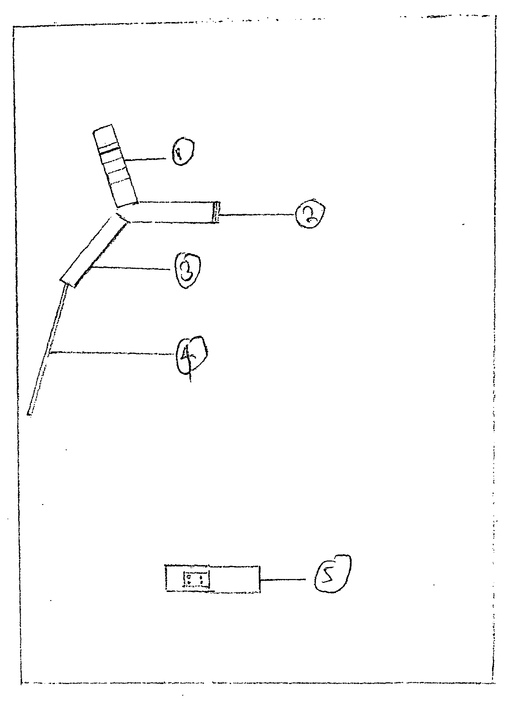

## Claims

1. Bladder sphincter pace maker gauging the bladder pressure and stimulating the fast acting sphincter muscle to contract and stop urine leakage.

2. Bladder sphincter pace maker, Radio frequency controlled activation of sphincter muscle relaxation to allow passage of urine.

3. Bladder sphincter pacemaker Pressure gauging adjustability.

4. As in Claim 1 Removable.
